**Europäisches Patentamt**

(19) **European Patent Office**

**Office européen des brevets**

(11) Numéro de publication: **0 161 267**
**B1**

(12) # FASCICULE DE BREVET EUROPÉEN

(45) Date de publication du fascicule du brevet:
22.07.87

(21) Numéro de dépôt: **84903711.4**

(22) Date de dépôt: **16.10.84**

(86) Numéro de dépôt international:
**PCT/CH 84/00168**

(87) Numéro de publication internationale:
**WO 85/01661 (25.04.85 Gazette 85/10)**

(51) Int. Cl.⁴: **A 61 M 16/00**

(54) **APPAREIL DE THERMOTHERAPIE PAR INHALATION.**

(30) Priorité: **20.10.83 CH 5708/83**

(43) Date de publication de la demande:
**21.11.85 Bulletin 85/47**

(45) Mention de la délivrance du brevet:
**22.07.87 Bulletin 87/30**

(84) Etats contractants désignés:
**AT BE CH DE FR GB IT LI LU NL SE**

(56) Documents cité:
**FR-A-2 267 831**
**FR-E-78 035**
**US-A-2 023 324**
**US-A-3 894 537**
**US-A-3 990 441**
**US-A-4 195 044**
**US-A-4 268 460**
**US-A-4 401 114**

(73) Titulaire: **CONSOL CORPORATION, 2-3 Kudan-
Minami 2-chome Chiyoda- ku, Tokyo 102 (JP)**

(72) Inventeur: **NOIR, Dominique, 21, Mont- Blanc, CH-
1170 Aubonne (CH)**
Inventeur: **TROUILHET, Yves, 4B, chemin des
Tattes, CH- 1222 Vesenaz (CH)**

(74) Mandataire: **Dousse, Blasco, 7, route de Drize, CH-
1227 Carouge/Genève (CH)**

**EP 0 161 267 B1**

LIBER, STOCKHOLM 1987

**Description**

La présente invention se rapporte à un appareil de thermothérapie par inhalation.

Les travaux du Professeur André Lwoff ont démontré que le développement d'un virus est fonction de la température, que l'on peut distinguer en trois zones: optimale, infra et supra-optimale. Aux températures supra-optimales, le matériel génétique viral est détruit par les enzymes lysozomiens précocement libérés et le développement du virus est bloqué. Ces travaux font l'objet d'un compte-rendu à l'Académie des Sciences de Paris, t.291 (8 déc. 1980) série D-957.

Sur la base de ces travaux, une équipe de chercheurs franco-israélienne des Instituts Weizmann et Pasteur a mis au point une thermothérapie du coryza infectieux et des rhinites persistantes allergiques. A cet effet, un appareil appelé "rhinotherme" a été conçu et a fait l'objet de US-A-4 401 114. Les essais cliniques basés sur cette thermothérapie ont permis d'entraîner une cessation prolongée des symptômes de la maladie chez un pourcentage élevé des malades notamment chez des malades atteints de rhinites persistantes rebelles à tout autre traitement.

Bien que ces résultats soient encourageants, la complexité et le coût de l'appareil mis au point à cet effet ne rendent pas cette thérapeutique accessible au grand public, de sorte que cette thérapeutique simple et entièrement naturelle n'est utilisée en pratique qu'en milieu hospitalier tout au plus. Or, il est évident qu'un appareil de conception plus simple, vendu à un prix abordable, trouverait naturellement sa place dans n'importe quelle pharmacie de ménage au même titre que les brosses à dents électriques ou autres appareils de soin des gencives. La large diffusion d'un tel appareil pourrait avoir des répercussions bénéfiques aussi bien sur les finances des assurances maladie que sur l'économie en général, compte tenu de la fréquence de ces affections virales, entraînant certains hivers la perte d'un nombre considérable de journées de travail.

Le but de la présente invention est précisément un appareil de thermothérapie par inhalation de conception simple, de fonctionnement sûr et dont le coût permet d'en faire un appareil à usage domestique.

A cet effet, la présente invention a pour objet un appareil de thermothérapie par inhalation selon la revendication.

La simplicité de cet appareil résulte de sa conception qui ne fait intervenir aucune pièce mobile, l'élément moteur étant constitué soit par la vapeur sous pression résultant du chauffage de l'eau et alimentant un venturi, soit par la dépression consécutive à une aspiration. Les paramètres de fonctionnement étant quasi constants, la température du mélange air-vapeur inhalé peut être fixée par construction.

Un appareil comprenant les éléments indiqués dans le préambule de ladite revendication est exposé dans le document US-A-2023324.

La particularité relative à la distribution de gouttelettes dont la taille est sensiblement supérieure à celles formées par la vapeur d'eau s'est révélée importante pour que l'appareil atteigne l'effet thérapeutique pour lequel il a été conçu. En effet, les gouttelettes trop fines tel qu'en produit la vapeur sont essentiellement inhalées jusque dans les poumons, de sorte que le chauffage des muqueuses à soigner est moins efficace. L'adjonction d'eau pulvérisée permet de former des gouttelettes plus grosses qui viennent mouiller la muqueuse, améliorent l'effet thermique recherché et accroissent l'efficacité du traitement.

Le dessin annexé illustre schématiquement et à titre d'exemple, deux formes d'exécution et une variante de l'appareil objet de la présente invention.

La figure 1 est une vue en élévation en coupe de l'une de ces formes d'exécution.

La figure 2 est une vue partielle en élévation d'une variante avec partie arrachée.

La figure 3 est un schéma de la seconde forme d'exécution.

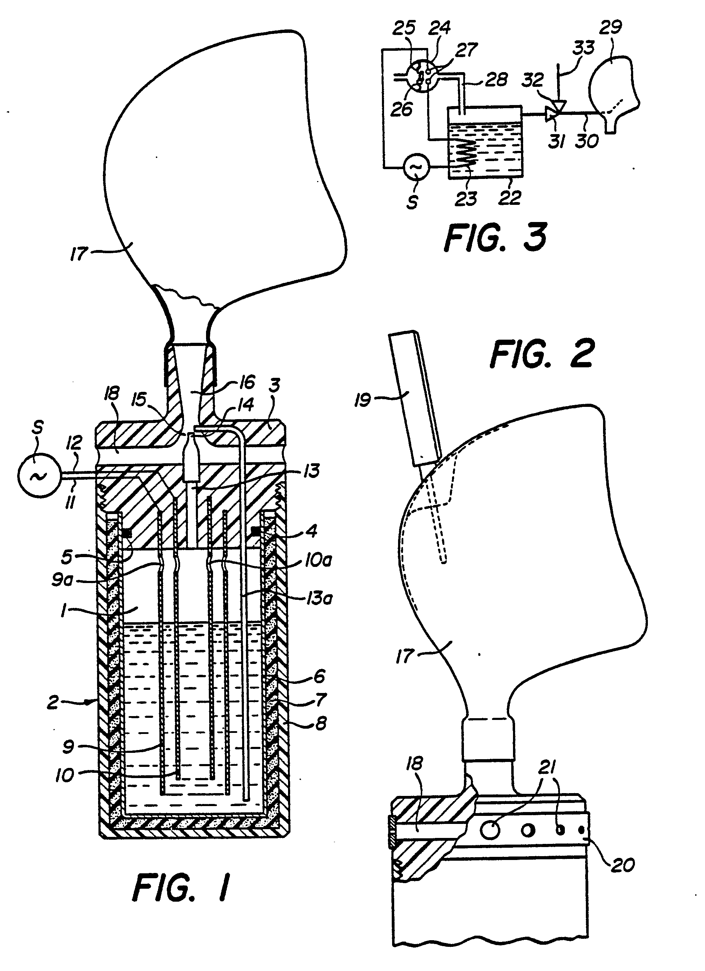

L'appareil illustré par la figure 1 comporte une enceinte 1 formée d'un récipient cylindrique 2 fermé par un bouchon 3 vissé sur ce récipient. Un joint d'étanchéité O-ring 4 disposé dans une gorge 5 ménagée dans la face latérale de ce bouchon 3 sert à réaliser l'étanchéité entre le bouchon 3 et le récipient cylindrique 2.

La paroi du récipient 2 comporte une partie intérieure 6 en acier inoxydable entourée d'une couche thermiquement isolante 7 et d'une enveloppe extérieure 8 qui peut être en matière plastique par exemple.

Le bouchon 3 porte, concentriquement à son axe de révolution, deux tubes coaxiaux 9 et 10 en acier inoxydable, en alliage inaltérable ou en titane platinisé par exemple. Ces tubes sont reliés par deux conducteurs respectifs 11 et 12 à une source de courant alternatif S, et présentent des perforations 9a, respectivement 10a, à leur partie supérieure. Ces tubes, qui servent d'électrodes de chauffage d'une eau non distillée, peuvent évidemment être remplacés par deux simples tiges (non représentées) qui se terminent avantageusement à leurs extrémités inférieures par deux sphères ou disques en vue d'augmenter la surface conductrice des électrodes dans le fond du récipient, de manière que le chauffage soit moins dépendant de la hauteur du niveau de l'eau, celle-ci constituant à la fois la résistance de chauffage et le produit distribué sous forme de vapeur et de gouttelettes.

Un conduit 13 traverse axialement le bouchon 3 et se termine par une buse 14 qui pénètre dans le col d'un venturi 15 formé à la jonction d'un conduit 16 débouchant dans un masque d'inhalation 17 et d'un conduit 18 traversant diamétralement le bouchon 3 pour faire communiquer le col du venturi 15 avec l'atmosphère. Un tube capillaire 13a relie le fond du récipient 2 à la buse 14. Il faut encore relever que le masque 17 constitue un exemple. Il pourrait être remplacé par deux conduits destinés à être logés directement dans les narines et à éviter de nuire à la peau du visage sous l'effet de la vapeur.

L'appareil décrit fonctionne de la façon suivante: de l'eau est versée dans le récipient 2 jusqu'à un niveau marqué sur la paroi intérieure 6 de ce récipient, ce niveau se trouvant à une certaine distance, par exemple 3 cm, de la face inférieure du bouchon 3. Le bouchon 3 avec ses électrodes tubulaires 9 et 10 est vissé sur le récipient 2 et les électrodes sont reliées à la source de courant alternatif S. Le passage du courant entre ces électrodes provoque le chauffage de l'eau. Les ouvertures 9a et 10a permettent de maintenir l'eau au même niveau dans les différentes zones du récipient 2 et la circulation de la vapeur vers le conduit 13. Lorsque l'eau bout après environ 1 à 2 min, la vapeur produite s'échappe sous pression à travers le conduit 13 et la buse 14 et crée une dépression au niveau du col du venturi 15 qui entraîne de l'air. Le réglage du venturi est fait en sorte que le mélange d'air ambiant à environ 20°C et de vapeur d'eau à 100°C donne de l'air pratiquement saturé d'eau à environ 43°C, avec un débit de l'ordre de 45 l/min. Ce débit constant représente 3/4 de litre par seconde qui correspond au volume inhalé par un individu adulte.

La vapeur d'eau arrive à la buse 14 avec une pression de l'ordre $10^3$ à $10^4$ Pa. La buse 14 présente un diamètre de 0,5 à 2 mm et la vitesse de la vapeur est de l'ordre de 50-200 m/s. Le débit de vapeur est de l'ordre de 0,33 m³/h avec une densité de 0,6 kg/m³. Si l'on tient compte du fait que le traitement dure une demi-hueure environ, la consommation d'eau est de l'ordre de 1 dl de sorte que la capacité de l'enceinte 1 est de l'ordre de 150 cm³.

L'air entraîné par la vapeur d'eau est l'air ambiant à environ 20°C à raison d'environ 2 m³/h soit environ 2,6 kg/h. Ceci donne un débit massique d'air saturé de vapeur d'eau à 43°C de 2,8 kg/h. En raison de la surpression régnant dans le récipient 2 et de la dépression régnant au niveau de la buse 14, une certaine quantité d'eau est pulvérisée à la sortie du conduit 13a sous la forme de gouttelettes de 2 à 20 µm qui sont destinées à se déposer sur la muqueuse nasale et à améliorer l'efficacité du chauffage de cette muqueuse qui doit être convenablement humidifiée.

La variante illustrée par la figure 2 comporte de plus un dispositif de mesure de la température, constitué par exemple par un thermomètre à bilame 19 disposé dans le masque d'inhalation 17 et dont le cadran d'affichage est à la vue de l'utilisateur pendant l'inhalation, ce qui lui permet de s'assurer du bon déroulement du traitement.

Ce dispositif de mesure est complété par un organe de réglage comprenant une bague 20 montée rotativement autour de la partie du bouchon 3 traversée par le conduit diamétral 18.

Cette bague 20 présente deux séries d'ouvertures 21 dont les diamètres sont décroissants dans le même sens. Les ouvertures de chaque série dont les diamètres correspondent sont diamétralement opposées autour de la bague 20, de sorte qu'ils peuvent être placés simlutanément vis-à-vis des deux extrémités du conduit diamétral 18. Grâce à cette bague de réglage 20, l'utilisateur peut intervenir au cas où la température de l'atmosphère créée à l'intérieur du masque d'inhalation 17 n'est pas correcte. Si cette température est trop élevée, il tournera la bague 20, de manière à disposer des ouvertures 21 de plus grands diamètres aux extrémités du conduit diamétral 18, de sorte que la proportion d'air entraîné par le venturi 15 augmentera et que la température du mélange air-vapeur d'eau diminuera. Dans le cas contraire, il disposera des ouvertures 21 de plus petits diamètres en face du conduit diamétral 18 et la température du mélange augmentera. En fait, l'appareil sera étalonné constructivement pour délivrer un mélange à 43°C à partir d'air à 20°C, ce qui représente le cas d'utilisation le plus fréquent. Toutefois, le dispositif de mesure et de réglage permet d'effectuer un contrôle et d'ajuster la température en cas de fonctionnement à partir d'autres conditions, lorsque la température ambiante diffère des 20°C, celle de la vapeur demeurant une constante.

L'appareil de thermothérapie par inhalation selon l'invention est donc extrêmement simple, ce qui permet d'en faire un appareil idéal à usage domestique, prenant ainsi place dans la pharmacie de ménage. Non seulement sa conception simple permet de le produire à un prix abordable, mais il ne nécessite pratiquement aucun entretien, si ce n'est des mesures élémentaires d'hygiène qui peuvent consister soit dans le nettoyage du masque 17, soit dans son remplacement après chaque usage ou après la série de traitements nécessaires pour guérir un même malade.

Pour chauffer l'eau à l'aide des électrodes décrites en utilisant la conductivité de l'eau, il y a lieu de prendre une eau du robinet dont la conductivité peut varier entre 1000/µS/cm et 4000 µS/cm.

Lorsque la conductivité de l'eau est trop faible pour la surface des électrodes, on peut l'augementer par l'adjonction d'un acide ou de NaCl.

Dans ce cas, la conductivité de la solution augmentera au fur et à mesure de l'évaporation de l'eau, puisque la concentration de la solution s'élèvera au fur et à mesure de cette évaporation. Etant donné que la surface d'électrodes immergée diminue avec l'abaissement du niveau de la solution, on peut obtenir une puissance sensiblement constante.

L'utilisation de l'eau du robinet ne pose pas de problème particulier en ce qui concerne les bactéries étant donné que cette eau est chauffée à sa température d'ébullition pour produire la vapeur nécessaire à la bonne marche de l'appareil. L'entartrage qui peut résulter de l'utilisation d'une eau dure peut être dissous par une solution d'acide acétique à 10%. L'utilisation d'eau distillée additionnée de NaCl constitue une mesure qui permet de supprimer le problème d'entartrange.

A titre d'exemple, le dimensionnement des électrodes peut être le suivant:

si l'on admet un débit d'air saturé de vapeur de 0,75 l/s correspondant à 2,86 kg/h, le débit massique d'eau selon le diagramme de Mollier sera:

M = 0,18 kg/h

la puissance électrique nécessaire est

$$Q = 0,18 \tfrac{kg}{h} . 2592 \tfrac{kJ}{kg} . \tfrac{h}{3600s} = 130 \ W$$

ce qui donne $I = \tfrac{130 \ W}{220 \ V} = 0,59 \ A$

la résistance devra être de

$$R = \tfrac{220V}{0,59A} = 372 \ ohms$$

$R = \tfrac{L}{\gamma S_e}$ où L = distance entre électrodes

γ = conductivité
$S_e$ = surface des électrodes

Etant donné que pour éviter l'effet d'électrolyse de l'eau la densité de courant $I/S \leqslant 1200 \ A/m^2$,

si $\gamma = 1000/\mu S/cm$, $1000 \tfrac{1}{M\Omega cm}$

$S_e = 5 \ cm^2$

$L = S_e \gamma R, 5.1000.10^{-6} \tfrac{1cm^2}{\Omega} \ cm \ 372 \ Ohms$

$L = 1,86 \ cm$

Le calcul du choix des dimensions doit tenir compte d'une diminution importante de la puissance dès le début de la vaporisation du fait de la présence des bulles de vapeur qui diminuent la surface mouillée utile des électrodes.

Bien entendu, outre la modification de l'acidité de l'eau, on peut également agir par exemple sur l'écartement entre les électrodes. Bien que le système de chauffage soit constitué avantageusement par deux électrodes connectées à une source de courant altenatif qui présente l'avantage d'être bon marché et d'éviter toute surchauffe accidentelle du fait de l'absence de chauffage en l'absence d'eau, on peut cependant envisager d'autres types de chauffage tel que celui par effet Joule qui consiste à plonger une résistace électrique dans l'enceinte 1 à la place des électrodes.

La seconde forme d'exécution illustrée très schématiquement par la fig. 3 comporte une enceinte 22 dans laquelle un corps de chauffe 23 plonge dans l'eau. Ce corps de chauffe, qui peut être composé de deux électrodes et d'une eau à faible conductivité comme dans l'exemple précedemment décrit, est relié à une source de courant S par un contacteur comprenant un boîtier 24 divisé en deux parties par une membrane élastique 25 portant une pastille de métal 26. L'un des conducteurs reliant la source de courant S au corps de chauffe 23 passe à travers le boîtier 24 et est interrompu entre deux bornes 27. La partie du boîtier 24 contenant les bornes 27 est connectée par un conduit 28 au sommet de l'enceinte 22, tandis que l'autre partie de ce même boîtier 24 est reliée à l'atmosphère de sorte que la position de la pastille de métal 26 par rapport aux bornes 27 est fonction de la différence de pression entre l'atmosphère et l'enceinte 22.

Le sommet de cette enceinte 22 est encore relié à un masque d'inhalation 29 par un conduit 30, le long duquel une valve unidirectionnelle 31 contrôle l'écoulement de la vapeur produite dans l'enceinte 22. Cette valve est normalement fermée lorsque la pression est égale en amont et en aval du conduit 30 et s'ouvre lorsque la pression est plus élevée en amont qu'en aval de manière à permettre à la vapeur de s'écouler en direction du masque d'inhalation 29. Une seconde valve 32 commande le passage d'un conduit 33 en communication avec l'atmosphère et débouchant dans le conduit 30 en aval de la valve 31. Cette valve 32 est identique à la précédente de sorte qu'elle s'ouvre également lorsque la pression régnant en aval de cette valve 32 est plus faible que la pression atmosphérique. Chaque valve 31, 32 peut être associée à des moyens de réglage de la section de passage des conduits respectifs 30 et 33.

La soconde forme d'execution comprend aussi un venturi et un second conduit semblable au tube capillaire 13a. Ce venturi et second conduit ne sont pas illustrés par la figure 3.

L'appareil conforme à cette forme d'exécution fonctionne de la façon suivante: lorsque la pression est identique des deux côtés de la membrane 25, celle-ci applique la pastille de métal 26 contre les bornes 27, mettant sous tension le corps de chauffe 23. Lorsque l'eau contenue dans l'enceinte 22 bout et produit de la vapeur, la pression monte légèrement et la membrane 25 écarte alors la pastille de métal 26 des bornes 27 coupant l'alimentation du corps de chauffe 23. L'appareil est alors prêt à fonctionner.

Dès qu'une dépression règne dans la partie aval du conduit 30 consécutivement à une aspiration exercée dans le masque, les valves 31 et 32 s'ouvrent simultanément, laissant alors passer de la vapeur et de l'air dans des proportions qui sont fonction de leurs sections de passage respectives.

Dès que la pression dans l'enceinte 22 diminue consécutivement à l'écoulement de vapeur vers le masque d'inhalation 29, le contact entre la pastille métallique 26 et les bornes 27 se rétablit et le chauffage de l'eau dégage à nouveau de la vapeur. Dès que l'utilisateur cesse son aspiration, les valves 31 et 32 se referment et la production de vapeur dans l'enceinte 22 cesse aussitôt que la pression dépasse légèrement la pression atmosphérique. Cette forme d'exécution diffère de la précédente essentiellement en raison du mode de fonctionnement discontinu, l'écoulement du mélange air-vapeur ne se produisant que consécutivement à une dépression dans le conduit 30, engendrée par l'aspiration que l'utilisateur exerce à l'extrémité amont de ce conduit. L'utilisation de deux électrodes et de la résistance de l'eau comme corps de chauffe présente l'avantage d'une inertie quasiment nulle, assurant un temps de réaction pratiquement instantané.

En variante, il est encore possible de supprimer le contact commandé par la membrane 25 et de relier la partie supérieure de l'enceinte 22 avec l'atmosphère par un évent calibré (non représenté) destiné à maintenir une pression sensiblement constante dans cette enceinte 22, cette pression étant choisie inférieure à la pression d'ouverture de la valve 31.

## Revendication

Appareil de thermothérapie par inhalation comprenant une enceinte (1) pour chauffer de l'eau à sa température d'ébullition, un conduit (13) dont une extrémité débouche dans la partie supérieure de cette enceinte (1) et dont l'autre extrémité (14) s'ouvre dans un canal (15, 16) présentant une admission (18) reliée à l'atmosphère, située en amont de l'autre extrémité (14) dudit conduit (13) et une sortie (17) située en aval, l'écoulement de la vapeur sous pression à la sortie (17) dudit conduit (13) entraînant un écoulement d'air à travers ledit canal (15, 16) formant un mélange d'air humide et chaud à sa sortie (17), caractérisé par le fait que l'extrémité (14) dudit conduit (13) qui débouche dans ledit canal (15, 16) est constituée par un éjecteur (14), la portion du canal recevant cet éjecteur formant le col d'un venturi (15), cet éjecteur (14) et ce venturi (15) étant dimensionnés pour former un mélange d'air humide dont la température est comprise entre 38°C et 50°C, et par le fait qu'un second conduit (13 a) présente une extrémité placée au-dessous du niveau de liquide de ladite enceinte (1) et l'autre extrémité adjacente audit éjecteur (14) pour alimenter ledit canal (15, 16) en gouttelettes dont la grosseur est comprise entre 2 et 20 μm.

## Patentansprüche

Vorrichtung zur Thermotherapie durch Inhalieren, mit einem Behälter (1) zur Erwärmung von Wasser auf seine Siedetemperatur, mit einer Leitung (13), deren eines Ende in den oberen Teil dieses Behälters (1) mündet und deren anderes Ende sich in einen Kanal (15, 16) öffnet, der einen mit der Atmosphäre verbundenen Zugang (18) vor dem anderen Ende der Leitung (13) und einen nachgeschalteten Austritt (17) darstellt, wobei der Strom des unter Druck stehenden Dampfes am Austritt (17) dieser Leitung (13) eine Luftströmung über den Kanal (15, 16) zufolge hat und am Austritt (17) eine Mischung feuchter und warmer Luft gebildet wird, dadurch gekennzeichnet, daß das Ende (14) dieser Leitung (13), die in den Kanal (15, 16) mündet, durch eine Düse (14) gebildet ist, wobei der diese Düse aufnehmende Kanalabschnitt den Hals eines Venturi (15) bildet, und Düse (14) und dieser Venturi (15) ausgebildet sind, um eine Mischung feuchter Luft zu bilden, deren Temperatur zwischen 38 und 50°C liegt, und dadurch gekennzeichnet, daß eine zweite Leitung (13a) ein Ende unterhalb des Flüssigkeitsniveaus des Behälters (1) und ein anderes Ende nahe dieser Düse (14) hat, um dem Kanal (15, 16) Tröpfchen zuzuführen, deren Größe zwischen 2 und 20 μm liegt.

## Claim

Apparatus for heat therapy by inhalation comprising an enclosure (1) for heating water at its boiling temperature, a tube (13) of which one end opens into the top part of this enclosure (1) and the other end (14) of which opens into a pipe (15, 16) provided with an inlet (18) connected to the atmosphere located upstream of said tube (13) other end (14) and an outlet (17) located downstream thereof, the flow of pressurized steam at the exit (17) of said tube (13) drawing an air current through said pipe (15, 16) forming a mixture of hot and moist air at the outlet (17) thereof, characterized by the fact that the tube (13) end (14) which opens into said pipe (15, 16) is constituted by a nozzle (14), the portion of the pipe housing this nozzle forming the neck of a venturi (15), this nozzle (14) and this venturi (15) being dimensioned to form a moist air mixture of a temperature comprised between 38 and 50°C, and by the fact that a second tube (13a) is provided with an end located below the level of the liquid in said enclosure (1) and the other end is adjacent to said nozzle (14) for supplying said pipe (15, 16) with droplets of size comprised between 2 and 20 μm.

0 161 267

25 24 27 29 33

26 28 32

S 31 30

23 22

**FIG. 3**

**FIG. 2**

17

18 16 3 19

18 15 14

S 12

11 13

5 17

9a 10a

1 13a 18 21

2 20

6

7

8 20

9

10

**FIG. 1**

1